Europäisches Patentamt

European Patent Office

Office européen des brevets

(19)

(11) Numéro de publication: **0 384 831**
**A2**

(12) ## DEMANDE DE BREVET EUROPEEN

(21) Numéro de dépôt: **90400464.5**

(51) Int. Cl.⁵: **A61B 17/22**

(22) Date de dépôt: **20.02.90**

(30) Priorité: **21.02.89 FR 8902250**

(43) Date de publication de la demande:
**29.08.90 Bulletin 90/35**

(84) Etats contractants désignés:
**AT BE CH DE DK ES GB GR IT LI LU NL SE**

(71) Demandeur: **TECHNOMED INTERNATIONAL S.A.**
**Le Ponant 1 11 rue Leblanc**
**F-75015 Paris(FR)**

Demandeur: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Cathignol, Dominique**
**14, rue du Fort**
**F-69740 Genas(FR)**
Inventeur: **Chapelon, Jean-Yves**
**6, Allée Marcel Achard**
**F-69100 Villeurbanne(FR)**

(74) Mandataire: **Portal, Gérard et al**
**Cabinet Beau de Loménie 55, rue d'Amsterdam**
**F-75008 Paris(FR)**

(54) **Appareil de destruction sélective de cellules incluant les tissus mous et les os à l'interieur d'un corps d'un être vivant par implosion de bulles de gaz.**

(57) L'invention concerne un appareil de destruction sélective de cellules (12) incluant les tissus mous et les os à l'intérieur du corps d'un être vivant.

Cet appareil est caractérisé en ce qu'il comprend :
- des moyens (20) de génération de bulles de gaz (22) in situ au sein des cellules à détruire sélectivement, et
- des moyens d'implosion (24), capables de réaliser une implosion in situ des bulles de gaz (22) qui résulte à une destruction des cellules (12) à détruire, adjacentes aux bulles de gaz implosées (22).

Grâce à l'invention, il est possible de détruire des cellules (12) à l'intérieur du corps d'un être vivant par voie non invasive, extracorporelle, d'une manière extrêment simple et efficace, permettant également de traiter des métastases.

EP 0 384 831 A2

## Appareil de destruction sélective de cellules incluant les tissus mous et les os à l'intérieur du corps d'un être vivant par implosion de bulles de gaz

La présente invention concerne essentiellement un appareil de destruction sélective de cellules incluant les tissus mous et les os à l'intérieur du corps d'un être vivant, en particulier un mammifère, incluant un être humain.

Dans l'état de la technique antérieure, on a déjà décrit divers dispositifs et procédés de destruction sélective de cellules à l'intérieur du corps d'un être vivant, en particulier d'un mammifère, incluant un être humain. Par exemple, le brevet US RIEBER 2,559,227 décrit un dispositif générateur d'ondes de choc permettant de détruire des cellules à l'intérieur du corps d'un être humain, utilisant un ellipsoïde tronqué fermé hermétiquement par une membrane. L'énergie qu'il est nécessaire d'utiliser avec cet appareil pour détruire les cellules est relativement élevée, ce qui n'est pas souhaitable car il se présente le risque de détruire des cellules saines.

Par ailleurs, on connaît depuis un certain nombre d'années divers procédés de diagnostic permettant de détecter de manière non invasive une hypertension pulmonaire ou la présence de tumeurs. Par exemple, TICKNER et al. ont décrit dans "non invasive Assessment of Pulmonary Hypertension Using the Bubble UTS Resonance Pressure (BURP) Method", Nat. Tech. Inf. Service Rept. n° HR-62917-1A, avril 1977, ainsi que notamment par US 4 265 251, un procédé de diagnostic utilisant l'injection de bulles de gaz ou de précurseur de bulles de gaz, dans des vaisseaux pour permettre de déterminer la pression sanguine grâce à un appareil de détection ultrasonique, pour déterminer le débit sanguin et en déduire des données permettant de diagnostiquer la présence ou l'absence d'une maladie cardiaque ou pulmonaire. Dans US 4 316 391, on décrit l'utilisation d'un matériau solide précurseur de bulles sous la forme de microcapsules encapsulant un gaz de composition choisie, ayant un diamètre compris entre 0,5 et 300 $\mu$m. Dans le document EP-A-0 072 330, on décrit des perfectionnements dans le système de détection ultrasonique dont on utilise des fréquences ultrasoniques pour générer des bulles fines in situ que l'on détecte par ailleurs par prise en compte de l'effet Doppler. On peut encore citer dans le même sens DE-A-29 46 662 et EP-A-0 273 140.

Par ailleurs, dans le document WO-A-80/02365 RAZOR, on utilise également l'injection de microbulles de gaz ayant un diamètre de 0,5 $\mu$m à 300 $\mu$m pour la détection de tumeurs ainsi que pour délivrer des agents thérapeutiques gazeux sélectivement à des tumeurs. (Voir page 4, lignes 4 à 9 et 14 à 30, ainsi que les revendications).

On conçoit ainsi que dans l'état antérieur de la technique, l'utilisation de bulles de gaz a été réalisée essentiellement ou uniquement dans un but de diagnostic, et surtout pour mesurer la pression sanguine. Seul le document WO-A-80/02365 prévoit la possibilité d'amener des agents thérapeutiques gazeux sélectivement aux tumeurs pour les traiter. Cette dernière solution n'est pratiquement pas envisageable puisque le nombre d'agents thérapeutiques qui peuvent être délivrés sous forme gazeuse et qui sont capables en outre de traiter une tumeur donnée, sont extrêmement réduits de sorte que ce procédé de traitement de tumeurs par un agent thérapeutique gazeux n'a pas débouché dans la pratique thérapeutique.

La présente invention a pour but de résoudre le nouveau problème technique consistant à trouver une solution qui permette d'utiliser des bulles de gaz pour réaliser in situ la destruction de cellules incluant les tissus mous et les os à détruire sélectivement.

La présente invention a encore pour but de résoudre ce nouveau problème technique par la mise en oeuvre d'une solution qui réalise cette destruction sélective des cellules principalement ou essentiellement par la mise en oeuvre de phénomènes physiques ou mécaniques, plutôt que par la mise en oeuvre de produits thérapeutiquement actifs dont la durée de traitement est longue dans le temps et nécessite des applications répétées.

La présente invention a encore pour but de résoudre le nouveau problème technique consistant en la fourniture d'une solution permettant de détruire sélectivement des cellules à l'intérieur du corps d'un être vivant, en particulier d'un mammifère, incluant un être humain, utilisant des effets physiques ou mécaniques combinés à l'introduction in situ au sein des cellules à détruire sélectivement de bulles de gaz, mais ne nécessitant si possible qu'un seul traitement, ou qu'un nombre extrêmement réduit de traitements, d'une manière non invasive, essentiellement extracorporelle.

La présente invention a encore pour but de résoudre ces nouveaux problèmes techniques d'une manière particulièrement simple, reproductible, extrêmement précise, c'est-à-dire d'une très grande sélectivité, utilisable à l'échelle industrielle.

La présente invention résoud pour la première fois ces problèmes techniques de la manière indiquée, ce qui constitue un progrès technique déterminant, totalement inattendu à l'homme de l'art, qui permet d'envisager de traiter un nombre considérables d'affections dues à des cellules malignes comme c'est le cas par exemple, à titre indicatif et non limitatif, des néoplasmes du foie, du pancréas, du rein, des testicules, de l'ovaire, de l'utérus, de la thyroïde, de la parathyroïde, du sein, des voies biliaires, de la peau, du sorrenace, du muscle, de la prostate, des glandes salivaires, des voies urinaires ; ou des os.

Ainsi, selon un premier aspect, la présente invention fournit un appareil de destruction sélective de cellules à l'intérieur du corps d'un être vivant, en particulier un mammifère, incluant un être humain, caractérisé en ce qu'il comprend :
- des moyens de génération de bulles de gaz in situ au sein des cellules à détruire sélectivement, et
- des moyens d'implosion capables de réaliser une implosion in situ des bulles de gaz qui résulte en une destruction des cellules adjacentes aux bulles de gaz implosées.

Selon un mode de réalisation avantageux de l'appareil selon l'invention, les moyens d'implosion précités comprennent des moyens de génération d'ondes acoustiques haute puissance.

Selon un mode de réalisation particulièrement avantageux, les ondes acoustiques précitées ont une onde de pression de quelques dizaines à quelques centaines de bars.

Selon un autre mode de réalisation particulièrement avantageux de l'invention, les moyens de génération d'ondes acoustiques précités comprennent un générateur acoustique du type transducteur piézoélectrique, de préférence comportant des moyens de focalisation des ondes acoustiques à un foyer cible.

Selon un autre mode de réalisation avantageux de l'invention, les moyens de génération d'ondes acoustiques précités comprennent un générateur d'ondes de choc mécanique, de préférence comprenant des moyens de focalisation des ondes de choc mécanique en un foyer cible. De tels appareils générateurs d'ondes de choc mécaniques comprennent des générateurs électrohydrauliques, des générateurs du type magnétostrictif, des générateurs à laser, des générateurs du type explosif.

Selon un mode de réalisation avantageux de l'appareil selon l'invention, les moyens de génération des bulles de gaz précités comprennent des moyens d'amenée de bulles de gaz in situ.

Selon une variante de réalisation, les moyens d'amenée de bulles de gaz in situ comprennent une solution injectable contenant des bulles de gaz.

Selon une autre variante de réalisation de l'invention, les moyens de génération de bulles de gaz comprennent un matériau précurseur de bulles de gaz. Selon une variante de réalisation, ce matériau précurseur de bulles de gaz comprend des capsules dans lesquelles les bulles de gaz sont emprisonnées, ces capsules étant réalisées en une matière se dissolvant progressivement dans le circuit sanguin. De tels matériaux sont décrits plus loin.

Selon un autre mode réalisation avantageux de l'appareil selon l'invention, les moyens de génération de bulles de gaz précités comprennent des moyens de cavitation générant des bulles de gaz in situ.

Selon un autre mode de réalisation avantageux de l'appareil selon l'invention, les moyens de génération de bulles de gaz précités comprennent un générateur ultrasonore à ondes négatives importantes. Des générateurs ultrasonores à ondes négatives importantes qui conviennent sont ceux pour lesquels l'onde négative peut atteindre une valeur négative de quelques bars à quelques dizaines de bars.

Selon une autre caractéristique avantageuse de l'appareil selon l'invention, celui-ci est caractérisé en ce qu'il comprend des moyens de repérage et de localisation des cellules à détruire, ainsi que des bulles de gaz.

Selon encore une autre caractéristique avantageuse de l'invention, le même générateur sert de moyen de génération de bulles de gaz et de moyen d'implosion de ces bulles de gaz.

Dans ce cas, ce générateur est un générateur d'ondes acoustiques de type ultrasonique.

Des appareils de génération d'ondes acoustiques haute puissance sont bien connus par le praticien. On peut utiliser tout générateur d'ondes acoustiques haute puissance utilisé pour la destruction de concrétion. De tels générateurs incluent des générateurs d'ondes acoustiques du type transducteur piézoélectrique actuellement disponible sur le marché. On peut également utiliser des générateurs d'ondes de choc mécaniques disponibles sur le marché, en particulier du type électrohydraulique pourvu de moyens de focalisation constitués par un ellipsoïde tronqué, de préférence celui commercialisé par TECHNOMED sous les dénomminations commerciales SONOLITH 2000® ou 3000®. On peut également utiliser des générateurs du type magnétostrictif disponibles sur le marché, en particulier ceux commercialisés par SIEMENS ou encore des générateurs d'ondes de choc mécaniques du type explosif (V DE-PS-2 351 247) ou à détonation (WO-87/03468), ou enfin des générateurs à laser (DE-2 538 260 ou EP-A-0 206 332). On peut encore citer le livre édité par Lawrence B.KANDLER Med. Doctor LLOYD H.HARRISON Med. Doctor, David C. MacCullough Med. Doctor en 1957, FUTURA Publishing Company, ayant pour titre "State of the art of extracorporeal Shockwave lithotrysy" qui décrit en détail les générateurs à microexplosion aux pages 111-145 (auteurs Mase Aki KUWAHARA et Kazu Yoshi TAKAYAMA) et les générateurs à laser (aux pages 97-110 (auteurs Michael E. MAYO et al.).

Pour les moyens de génération de bulles de gaz, on peut injecter des microcapsules précurseurs de bulles de gaz ayant de préférence un diamètre compris entre 0,5 μm et 300 μm, sans limitation, encapsulant un gaz de composition choisie, comme décrit dans le document WO-A-80/02365, que l'on injecte dans le circuit sanguin, soit par voie intraveineuse, soit par voie artérielle au niveau général ou au niveau irriguant les cellules à détruire. Ce matériau encapsulant les bulles de gaz est de préférence choisi par de la gélatine ou du saccharose. Le gaz utilisé peut être de l'azote ou du gaz carbonique. De telles microbulles peuvent être produites conformément au procédé décrit dans le document WO-A-80/02365 de la page 8, ligne 32, à page 10, ligne 12, qui est incorporé ici par référence et qui comprend l'écoulement du gaz à encapsuler à travers un petit orifice, par exemple à travers un tube capillaire, et dans un liquide gélifiable, comme la gélatine qui présente l'avantage bien connu d'être non toxique, non antigénique et non allergénique.

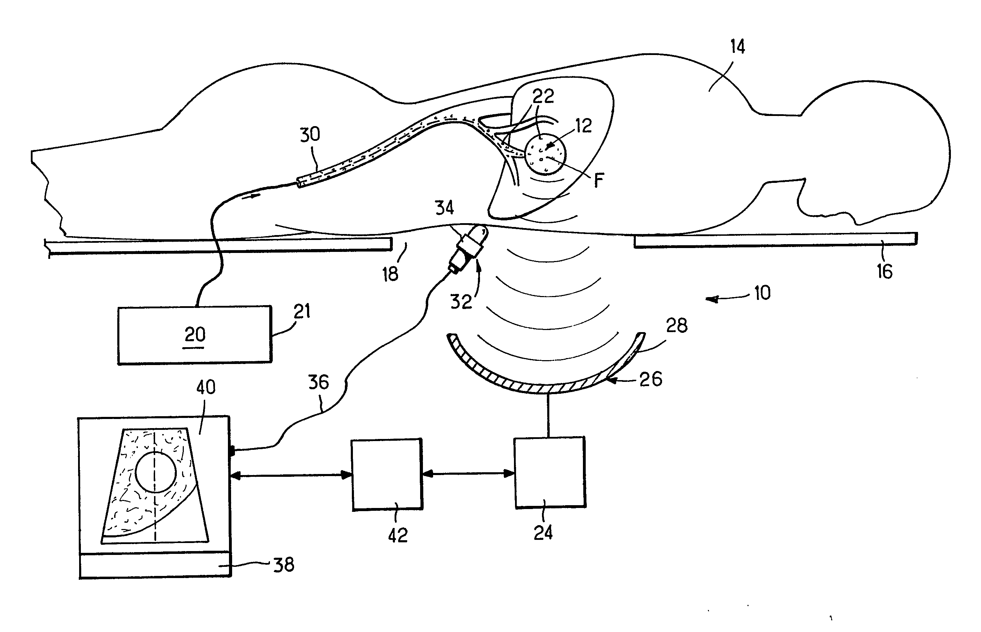

On peut également utiliser un matériau solide précurseur de bulles, qui libérera des bulles de gaz lorsqu'il est injecté dans le circuit sanguin comme décrit dans le brevet US-4 265 251 de la colonne 2, ligne 65, à colonne 4, ces bulles de gaz ainsi libérées dans le circuit sanguin étant aisément détectables par des moyens de détection ultrasoniques. On peut utiliser des particules ou microcapsules comprenant un espace intérieur creux, et dont les parois sont formées avantageusement à partir d'une composition consistant d'approximativement 80 % de saccharose et 20 % de lactose, l'espace creux à l'intérieur des capsules étant rempli d'un gaz à une pression supérieure à la pression qui existe dans le système cardiovasculaire. De telles capsules ou microcapsules encapsulant un gaz peuvent être préparées selon le procédé décrit dans le brevet US-3 012 893 et présentent l'avantage d'encapsuler des quantités sensiblement uniformes de gaz. On peut utiliser en particulier le dispositif décrit et représenté en référence à la figure 3, et encapsulant du dioxyde de carbone. On peut obtenir par ce procédé des microcapsules ayant un diamètre compris entre 0,5 et environ 350 μm qui génèreront des microbulles ayant un diamètre compris entre environ 1 μm et 150 μm.

D'autres matériaux pouvant servir pour la fabrication des parois des microcapsules encapsulant un gaz, sont décrits dans EP-A-123 235 , EP-A-131 540 ou encore EP-A-0 273 140. Comme matériaux des parois des microcapsules, on peut citer plus particulièrement la lécithine, des esters d'acides gras polyoxyéthylénés, des oléates de glycérine polyéthylèneglycol ricine, des polymères de polyoxyéthylènepolyoxypropylène, des esters de saccharose, des xyloglycérides, des alcools gras insaturés ($C_4$-$C_{20}$), des acides gras insaturés ($C_4$-$C_{20}$), des mono-, di- et triglycérides, des esters d'acides gras, constituant en particulier de 0,01 à 10 % en poids de la solution injectable. On peut encore citer la cyclodextrine, un monosaccharide, disaccharide ou trisaccharide, des polyols ou des sels minéraux ou organiques à une concentration de 5 à 50 % en poids. On peut encore citer le maltose, le dextrose, le lactose ou le galactose. Ces composés peuvent être en solution aqueuse en particulier des solutions physiologiques et notamment des solutions aqueuses à 0,9 % de NaCl. On peut également additionner ces solutions de composés augmentant la viscosité comme les monopolysaccharides, tels que le glucose, le lévulose, le galactose, le lactose, le sorbitol, le mannitol, le saccharose, le dextrane, la cyclodextrine, des polyols comme la glycérine ou des polyglycols. Des substances augmentant la viscosité comprennent également des protéines, des substances semblables aux protéines, des aminoacides, des substituts du sang comme des protéines du plasma, de la gélatine, des dérivés de gélatine ou leurs mélanges. La concentration peut varier de 0,5 à 50 % en poids, la concentration supérieure étant limitée par la capacité de solubilité de la substance. On peut également ajouter dans la solution des agents tensioactifs qui ont également un effet d'augmentation de viscosité comme les polymères de polyoxyéthylène et de polyoxypropylène dont le poids moléculaire peut être compris entre environ 4 500 et 16 500 en une proportion de 1 % à 20 %, et de préférence d'environ 3 % à 10 %.

De telles microcapsules sont aisément repérables par des moyens de repérage et de localisation de type ultrasonique fontionnant à une fréquence comprise entre quelques centaines de MHz et une dizaine de MHz.

Les bulles de gaz peuvent également être créées ou générées in situ grâce à un phénomène de cavitation réalisé à l'aide d'un générateur d'ondes ultrasoniques travaillant dans une zone de fréquence d'environ $10^4$ à $10^5$ Hz, ces ondes ultrasoniques ayant ainsi une durée d'environ une fraction de seconde à quelques secondes suivant l'énergie de l'onde, qui est nécessaire pour la formation des nucléies et la croissance des bulles in situ dans les fluides biologiques environnant les cellules à détruire, en particulier dans le sang des vaisseaux, incluant les capillaires, environnant les cellules à détruire.

D'autres buts, caractéristiques et avantages de l'invention apparaîtront clairement à la lumière de la description explicative qui va suivre faite en référence à la figure unique annexée représentant schématiquement et en coupe partielle un mode de réalisation actuellement préféré d'un appareil de destruction sélective de cellules à l'intérieur du corps d'un être vivant, selon l'invention, donnée simplement à titre d'illustration et qui ne saurait dont en aucune façon limiter la portée de l'invention.

En référence à la figure unique annexée, on a représenté un appareil actuellement préféré selon l'invention, représenté par le numéro de référence général 10, de destruction sélective de cellules représentées symboliquement par le chiffre de référence général 12, situées à l'intérieur du corps d'un être vivant 14, en particulier un mammifère, ici un être humain, qui repose par exemple sur une table support 16 comportant une ouverture 18 pour permettre le traitement de destruction sélective des cellules 12.

Cet appareil 10 selon l'invention est caractérisé en ce qu'il comprend :

a) des moyens, représentés par le numéro de référence général 20, de génération de bulles de gaz 22 in situ, au sein des cellules 12 à détruire sélectivement ; et

b) des moyens, représentés par le numéro de référence général 24, d'implosion, capables de réaliser une implosion in situ des bulles de gaz 22, qui résulte en une destruction des cellules 12 à détruire, adjacentes aux bulles de gaz implosées 22.

Les moyens 24 d'implosion des bulles de gaz 22 sont constitués avantageusement par des moyens de génération d'ondes acoustiques haute puissance qui, de préférence, génèrent des ondes de pression de quelques dizaines à quelques centaines de bars.

Selon un mode de réalisation particulier, les moyens 24 d'implosion comprennent un générateur acoustique du type transducteur piézoélectrique, de préférence comprenant des moyens 26 de focalisation en un foyer cible F, comprenant par exemple une surface de focalisation 28 de type hémisphérique, comme cela est bien connu à l'homme de l'art. De tels générateurs acoustiques du type transducteurs piézoélectriques focalisants sont disponibles sur le marché, en particulier pour réaliser la destruction de concrétion, notamment des lithiases rénales ou biliaires.

Selon un autre mode de réalisation particulier de l'invention, les moyens d'implosion 24 comprennent un générateur d'ondes de choc mécanique. Ces ondes de choc mécaniques peuvent être générées par un générateur de type électrohydraulique, de préférence comprenant des moyens de focalisation 26 comprenant un éllipsoïde tronqué. De tels appareils sont bien connus à l'homme de l'art et disponibles sur le marché. On peut citer avantageusement les appareils de TECHNOMED commercialisés sous la dénomination commerciale SONOLITH 2000® ou 3000® et utilisés jusqu'à présent pour la destruction de concrétions, en particulier des lithiases rénales ou biliaires.

On peut également utiliser des générateurs d'ondes de choc mécaniques du type magnétostrictif qui sont également bien connus à l'homme de l'art et disponibles sur le marché, en particulier les appareils commercialisés par la Société SIEMENS.

On peut également utiliser des générateurs d'ondes de choc mécaniques à laser qui sont également bien connus à l'homme de l'art.

On peut enfin utiliser des générateurs d'ondes de choc mécanique du type explosif, par exemple comprenant l'explosion d'un fil explosif comme cela est encore bien connu à l'homme de l'art.

Les moyens (20) de génération de bulles de gaz (22) selon l'invention ont été décrits en détail dans la partie introductive de la presente description. A titre d'exemple, il peut s'agir d'une solution injectable contenue dans un récipient 21 que l'on injecte dans le circuit sanguin, soit par voie intraveineuse, soit par voie artérielle 30, au niveau général ou au niveau irriguant les tissus 12 à détruire, comme cela est clairement représenté et compréhensible à l'homme de l'art à partir de la considération de la figure unique annexée. Ces bulles de gaz peuvent être des bulles d'azote, de dioxyde de carbone, ou d'un gaz inerte, tels que ceux qui sont utilisés dans les bouteilles de plongée sous-marine comme par exemple l'hélium.

Selon une autre caractéristique avantageuse de l'appareil selon l'invention, celui-ci comprend en outre des moyens 32 de repérage et de localisation des cellules 12 à détruire. Ces moyens de répérage 32 peuvent être avantageusement constitués par une sonde ultrasonique 34 auxiliaire, reliée par exemple par un conducteur 36 à un dispositif 38 de formation d'image sur un écran 40.

On peut également prévoir un dispositif de commande centrale 42 recueillant les données du dispositif 38 et commandant les moyens d'implosion 24 en fonction des données ainsi recueillies. Les appareils de génération d'ondes acoustiques haute puissance disponibles sur le marché comprennent en général tous des moyens 32 de répérage et de localisation auxiliaires ainsi que des moyens de contrôle 42 permettant de réaliser une corrélation entre le repérage et le positionnement de la cible à détruire 12 au point focal F des moyens de génération des ondes acoustiques 24-26 focalisées au point focal F.

En général, la destruction des cellules 12 à détruire, telles que des tumeurs, c'est-à-dire des cellules de tissu mou, ou des liaisons nerveuses, est obtenue à l'aide d'un traitement avec des ondes acoustiques générées par les moyens d'implosion 24-26, ayant des valeurs de pression de +300 bar pour l'onde positive et de -100 bar pour l'onde négative.

Par exemple, une destruction complète d'une tumeur du foie d'un lapin in vivo est obtenue avec l'application de 300 à 500 ondes élémentaires avec une onde ayant un temps de montée d'approximativement 100 ns et un temps de descente de 1 μs.

L'organe à traiter, par exemple ici le foie, est préalablement localisé par tout moyen de repérage et de localisation connu, par exemple par sonde ultrasonore 34, par rayons X, par RMN ou tout autre moyen de détection disponible à l'homme de l'art. Un système de mise en concordance des moyens de repérage 32 et des moyens d'implosion 24 est avantageusement prévu, comme cela est bien connu à l'homme de l'art et rappelé dans la description précédente.

Il est à noter que les bulles de gaz 22 qui sont introduites in situ, par l'un quelconque des moyens précédemment décrits, seront aisément visualisées par tout moyen ultrasonore car les bulles augmentent le contraste par ce moyen de repérage.

On peut ainsi constater que l'appareil selon l'invention permet de réaliser de nouveaux traitements thérapeutiques, d'une manière extrêmement simple, extracorporelle, car cette technique permet d'envisager la destruction de très petites tumeurs dès que les ondes sont focalisées dans un volume égal au volume à détruire. On peut ainsi détruire un volume aussi petit que 1 mm³ avec des ondes acoustiques de type ultrasonique et des tumeurs ayant un volume aussi réduit que 1 cm³ avec des ondes de choc mécaniques, en particulier générées par des moyens électrohydrauliques.

L'appareil selon l'invention permet donc de traiter également des métastases.

L'appareil selon l'invention permet de mettre en oeuvre de manière très souple de tels traitements thérapeutiques en raison du fait que les moyens d'implosion 24 peuvent être fournis par de nombreux appareils différents présentant individuellement des avantages particuliers qui peuvent être intéressants pour un traitement particulier.

En outre, comme cela a été décrit précédemment, les bulles de gaz peuvent également être amenées in situ selon de nombreuses manières différentes. Egalement, le diamètre de ces bulles de gaz peut varier dans de larges limites. Une limite supérieure réside dans le fait qu'un diamètre trop grand de ces bulles présente des risques d'embolisation pour les organes adjacents, tandis que lorsque ces bulles sont trop petites, il y a un risque pour que ces bulles de gaz soient éliminées avant avant même d'avoir pu réaliser le traitement. On préfère donc utiliser des bulles de gaz très stables comme celles qui ont été décrites précédemment et qui ont un diamètre qui varie de 0,1 à environ 300 μm.

Enfin, on peut observer que l'on peut utiliser le même générateur pour créer des bulles de gaz in situ grâce à des phénomères de cavitation, ou pour réaliser la destruction des cellules 12 à détruire au point focal F. Dans le premier cas, le générateur travaillera à faible puissance pour passer dans un deuxième stade à l'émission d'ondes de haute puissance capables de faire imploser les bulles de gaz créées par les phénomères de cavitation obtenus avec les ondes de faible puissance. Ceci sera en particulier très aisé avec des générateurs de type ultrasonique utilisant des éléments piézoélectriques. Avec ces appareils, en travaillant à quelques dizaines de kHz, on créera des bulles de gaz in situ grâce au phénomène de cavitation, tandis qu'en travaillant à plusieurs centaines de kHz à quelques MHz, on obtiendra l'implosion de ces bulles de gaz et la destruction des cellules 12 environnantes, se trouvant au foyer F.

L'invention concerne encore un procédé de traitement thérapeutique pour la destruction sélective des cellules à l'intérieur du corps d'un être vivant, en particulier un mammifère, incluant un être humain, caractérisé en ce qu'on génère des bulles de gaz in situ au sein des cellules à détruire sélectivement et on fait imploser ces bulles de gaz, ladite implosion résultant en une destruction des cellules à détruire adjacentes aux bulles de gaz implosées.

Des modes de réalisation particuliers de ce procédé résultent clairement de la description précédente pour l'homme de l'art.

Par ailleurs, la présente invention concerne encore un procédé de traitement de métastases, caractérisé en ce qu'on génère des bulles de gaz au sein ou à proximité immédiate des métastases et on provoque l'implosion de ces bulles de gaz, en obtenant ainsi une destruction des métastases.

On conçoit ainsi que l'invention n'est pas limitée aux modes de réalisation décrits. Au contraire, l'invention comprend tous les moyens techniques constituant des équivalents techniques des moyens décrits ainsi que leurs diverses combinaisons.

## Revendications

1. Appareil (10) de destruction sélective de cellules (12) à l'intérieur du corps d'un être vivant (14), en particulier un mammifère, incluant un être humain, caractérisé en ce qu'il comprend :
- des moyens (20) de génération de bulles de gaz (22) in situ au sein des cellules à détruire sélectivement, et
- des moyens d'implosion (24), capables de réaliser une implosion in situ des bulles de gaz (22) qui résulte en une destruction des cellules (12) à détruire, adjacentes aux bulles de gaz implosées (22).

2. Appareil selon la revendication 1, caractérisé en ce que les moyens d'implosion (24) précités comprennent des moyens (26) de génération d'ondes acoustiques haute puissance.

3. Appareil selon la revendication 2, caractérisé en ce que les ondes acoustiques haute puissance ont une onde de puissance de quelques dizaines à quelques centaines de bars.

4. Appareil selon la revendication 2 ou 3, caractérisé en ce que les moyens de génération d'ondes acoustiques précités comprennent un générateur acoustique du type transducteur piézoélectrique.

5. Appareil selon l'une des revendications 2 ou 3, caractérisé en ce que les moyens de génération d'ondes acoustiques précités comprennent un générateur d'ondes de choc mécaniques, en particulier du type électrohydraulique, magnétostrictif, à laser, ou du type explosif.

6. Appareil selon l'une des revendications 1 à 5, caractérisé en ce que les moyens de génération de bulles de gaz précités (20) comprennent des moyens (30) d'amenée de bulles de gaz (22) in situ.

7. Appareil selon la revendication 6, caractérisé en ce que les moyens (30) d'amenée de bulles de gaz comprennent un réservoir (21) d'une solution injectable contenant des bulles de gaz.

8. Appareil selon l'une des revendications 1 à 6, caractérisé en ce que les moyens de génération de bulles de gaz (20) précités comprennent un matériau précurseur de bulles de gaz.

9. Appareil selon la revendication 8, caractérisé en ce que le matériau précurseur de bulles de gaz précité comprend des capsules ou microcapsules dans lesquelles les bulles de gaz sont emprisonnées, la paroi des capsules ou microcapsules étant réalisée en une matière se dissolvant progressivement dans le circuit sanguin.

10. Appareil selon la revendication 9, caractérisé en ce que la paroi des capsules ou microcapsules est réalisée en gélatine, lécithine, des esters d'acides gras polyoxyéthylénés, en oléate de ricine de glycérine de polyéthyléneglycol, des polymères de polyoxyéthylénepolyoxypropyléne, un ester de saccharose, un alcool gras insaturée en $C_4$-$C_{20}$, un acide gras insaturé en $C_4$-$C_{20}$, un monoglycéride, un diglycéride ou un triglycéride, le maltose, le dextrose, le lactose ou le galactose.

11. Appareil selon l'une des revendications 1 à 6, caractérisé en ce que les moyens de génération de bulles de gaz précités (20) comprennent des moyens de cavitation (26) générant des bulles de gaz in situ par des phénomènes de cavitation.

12. Appareil selon la revendication 11, caractérisé en ce que les moyens de cavitation précités (26) comprennent un générateur ultrasonore à ondes négatives importantes pouvant aller de quelques bars à quelques dizaines de bars.

13. Appareil selon l'une des revendications 1 à 12, caractérisé en ce qu'il comprend des moyens (32) de répérage et de localisation des cellules (12) à détruire et des bulles de gaz (22), en particulier comprenant une sonde ultrasonique (34) ou un appareil à rayons X ou à RMN.

14. Appareil selon l'une des revendications 1 à 13, caractérisé en ce qu'il comprend une commande centrale (42).